# EUROPEAN PATENT SPECIFICATION

(11) **EP 0 613 385 B1**
(45) Date of publication and mention of the grant of the patent: **16.04.1997**
(21) Application number: 92923402.9
(22) Date of filing: 13.11.1992
(51) Int. Cl.: A61M 15/00

(54) **ORAL INHALER**
ORALER INHALATOR
INHALATEUR UTILISANT LA VOIE ORALE

(30) Priority: 18.11.1991 GB 9124468
(43) Date of publication of application: 07.09.1994
(73) Proprietor: SMITHKLINE BEECHAM PLC, Brentford, Middlesex TW8 9EP (GB)
(72) Inventor: GRATTAN, Timothy James, Weybridge, Surrey KT13 0DE (GB); WRIGHT, Davide John, SmithKline Beecham Pharmac., Epsom, Surrey KT18 5XQ (GB)
(74) Representative: Walker, Ralph Francis, Dr.
(86) International application number: GB9202113
(87) International publication number: WO9309831

(56) References cited:
- EP-A- 0 211 595
- FR-A- 2 133 122
- US-A- 1 985 001
- US-A- 2 318 636

## Description

This invention relates to a novel device, being an oral inhaler by means of which a volatile material may be inhaled.

Oral inhalers of this general type are known, for example as used by asthma sufferers etc. for the inhalation of a medicament as a vapour or as a stream of air-carried particles or droplets. It is desirable that such inhalers are convenient enough to be easily carried, for example in a pocket or handbag etc., for use when needed, that they should be cheap and robust, and that they should be capable of being closed so as to restrict ingress of contamination, or evaporation of volatile contents. One known type of inhaler is that described in ES-1007117-U (Calmante Vitaminado S.A.).

US-A-2318636 forming the preamble of claim 1 discloses an oral inhaler in which inhalation openings in a front wall of the inhaler body are closed by a hinged cover which is operated by a casing which slides parallel to a side wall of the body.

The present invention provides an oral inhaler which to some extent at least provides these desirable features in a particularly convenient form of device.

Accordingly this invention provides an oral inhaler comprising the subject matter of claim 1.

The side walls are suitably parallel and planar, and are suitably rectangular in shape. The term "rectangular" used herein includes "square", and also includes shapes which are generally rectangular but have rounded or chamfered corners, but which do not detract from the generally rectangular shape, for example lozenge shapes.

The box member is preferably tetragonal in general shape, although its walls may meet its edges with sharp corners, rounded corners or chamfered corners which themselves may meet the walls and edges with curves. In such a tetragonal box member the front edge, and the opposing edge may for example be profiled though without detracting from the overall generally tetragonal shape,. For example these edges may be curved, so that a cross section through the walls and these edges may be lozenge-shaped.

Suitably the edge distance is substantially less than the length and width dimensions of the side walls. Suitably the edge distance may be 0.25 or less, e.g. 0.2 or less, e.g. 0.1 or less of the length dimension of the box member. Suitable ratios of side wall length: side wall width: edge distance of the box member are 6-10 : 6-10 : 1. A suitable edge dimension for the box member is around 5 +/- 2 mm.

The front edge may be completely open, or it may comprise an edge wall having one or more orifices therein, of suitable dimensions to allow an easy inhalation of air without undue sucking effort. These openings may suitably total about 20 - 50 mm² in area. The edge opposing the front edge, and the remaining edges, i.e. in a tetragonal box member the edges at right angles to the front edge, i.e. end edges, may be closed, or may be partly or completely open.

The additional opening for the ingress of air, i.e. during the inhalation of air through the opening(s) in the front edge may be located in any position in the box member. For example the additional openings may be provided as openings in a rear edge opposing the front edge across the width of the box member, or in the side walls, or in the ends. The additional openings may for example be in one or both of the end edges of the box member, which may for example be completely open, however in such an embodiment the conformity of the box member and the casing should not be such as to restrict the flow of ingress air between the box member and the casing.

The substantially conforming casing may also be tetragonal, and may optionally have rounded or chamfered edges and/or corners, e.g. for safety (i.e. avoiding sharp corners) or for aesthetic appearance.

Suitably the box member may be completely contained within the casing when the casing is slid into its first position, and partly contained when the casing is slid into its second position. In this way the casing can prevent evaporation of the volatile material and contamination.

The casing is suitably slideably mounted such that it may slide in a direction parallel to the front edge. Slideable mounting may be achieved by the use of a casing which conforms very closely to the box member, so that there is some degree of friction between the box member and the casing, so that when the casing has been slid into the first or second position, or into an intermediate position there is some frictional resistance to sliding. This resistance can help to retain the casing in a desired position on the box member.

The surfaces of the box member, and the casing, e.g. the walls and edges which slide across each other may be provided with guide means, for example to assist smooth sliding and to help prevent jamming for example if the box member and the casing should twist out of alignment during sliding. Suitably the guide means may comprise interlocking ridges and corresponding grooves on the box member and the casing, running parallel to the direction of sliding. The extent of such interlocking may help in providing the above - mentioned degree of friction between the box member and the casing.

The box member and casing may be provided with end stop means to prevent the casing from being easily removed from the box member, e.g. to prevent it falling off. Suitable end stop means may comprise one or more grooves in one or more walls of the box member and/or casing, into which one or more cooperating projections on the casing and/or box member fit. Such groove(s) may be aligned parallel to the front edge. If such grooves do not run for the entire length of the wall(s) so that the projection(s) is/are only allowed a limited amount of travel, the end of the groove(s) may function as an end stop means. Conveniently if the groove(s) is/are in the form of slot openings in the wall(s) of the box member, it/they may comprise the additional opening(s) for the ingress of air. Other end stop means will be apparent to those skilled in the art, for example projections on the box member which may end stop against corresponding projections on the casing.

The casing may comprise a single casing element slideably mounted on the box member. In its first, covering, position such a single element may butt up against a flange on the box member to provide sealing against evaporation and contamination.

Alternatively the casing may comprise two casing elements slideably mounted on the box member. In their first, covering, position these two elements may butt up against each other or may fasten together, e.g. in a snap-fit or frictional interlocking, suitably meeting around the mid point of the length of the box member. The two elements may be independently slideable toward opposite ends of the box member, so that in the second, uncovered position the opening(s) in the front edge are located between the two casing elements.

The use of two casing elements in the above-described manner is particularly convenient, as the two casing elements, when in their second position, may be held by the fingers, and may thus form a handle, allowing the user to hold the inhaler in a manner similar to the use of a "mouth organ" or harmonica. This manner of use conveniently avoids the possibility of contamination of the vicinity of the front edge by contact with the hands.

The casing may be made of a size only slightly larger than the box member, so that the entire inhaler may be made in a "credit card" size and shape. Conveniently the entire box member and casing may be made of plastics materials such as polystyrene, polythene, or polypropylene.

An inhaler of the above-described type has the further advantage that in contrast to many present oral inhalers there is no separate removeable cover for the inhalation openings, which may easily be lost on opening, or suffer contamination if put down somewhere.

The volatile material for inhalation may comprise a known material for the easing of various respiratory symptoms, for example menthol. The material may be present as a solid, e.g. a pellet or encased within an air-permeable container or capsule, or may be a liquid, such as terpenoid oils, which may be impregnated on a wicking material or into a solid matrix.

To assist introduction of the volatile material into the box member, the latter may be made in an openable form, so that it can be manufactured in an open form, the volatile material inserted, and the box member then closed before insertion into the casing. When the volatile material is in the form of a liquid impregnated into a pad of wicking material, the box member may include internal positioning means to ensure that the pad is securely and correctly located within the box member.

The invention will now be described by way of example only with reference to the following drawings, in which:
Fig. 1 shows an overall perspective view of an inhaler with its casing in the second position.
Fig. 2 shows an overall perspective view of an inhaler with its casing in the first position.
Fig. 3 is a sectional view through the inhaler about the line A-A of Fig. 1 looking in the direction of the arrows.
Fig. 4 is a section through the inhaler of Fig. 1 about the line B-B.
Fig. 5 shows an overall perspective view of an alternative form of inhaler.
Figs. 6 and 7 show overall perspective views of alternative forms of box member.
Fig 8. shows a part sectional view of an inhaler of the invention incorporating a box member as shown in Fig 7.

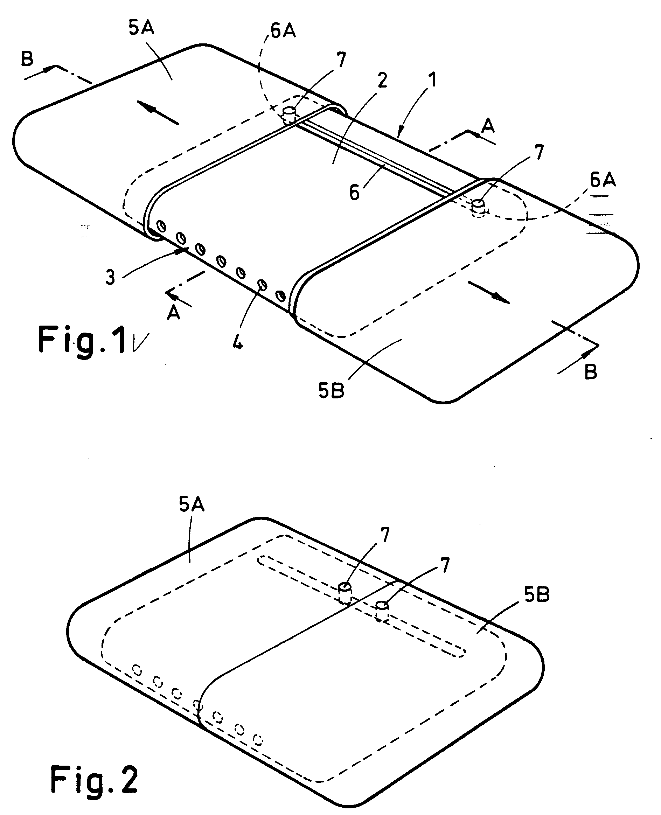

Referring to Fig. 1 an inhaler is shown overall. The inhaler comprises a box member (1), having two parallel, planar, rectangular walls (2), and being of a tetragonal shape. The box member (1) has a front edge (3), in which there are a number of openings (4) in the form of circular holes.

The box member (1) is partly contained within a casing in the form of two casing elements (5A,5B), and the part of the box member (1) which is partly contained is shown in Fig. 1 by dotted lines. Elements (5A,5B) are slideably mounted on box member (1) and are capable of sliding movement in the direction shown by the arrows. The box member (1) has slots (6) in its walls (2) which pass completely through the wall(s) (2), and which are parallel to the front edge (3) but do not run for the entire length of wall (2), terminating at slot ends (6A). The elements (5A,5B) have pins (7) projecting inwardly and which fit into slots (7). The pins (7) are for clarity shown as projecting through the wall of the elements (5A,5B), but are conveniently integrally moulded with elements (5A,5B). The elements (5A,5B) are thereby permitted to slide into the position shown in Fig. 1, but not to easily be separated from box member (1), by means of the end-stop interaction between pins (7) and slot ends (6A). The slots (6) serve an additional function of forming additional openings for the ingress of air during inhalation through openings (4).

Referring to Fig. 2, the inhaler of Fig. 1 is shown with the casing (5) in its first, covering, position, in which elements (5A,5B) have been slid together to meet at approximately the mid point of the length of walls (2), and to cover openings (4). The casing (5) substantially conforms in size and shape to the box member (1), and is of generally tetragonal shape but having rounded corners. The box member (1) contained within the casing (5) is shown with dotted lines in Fig. 2.

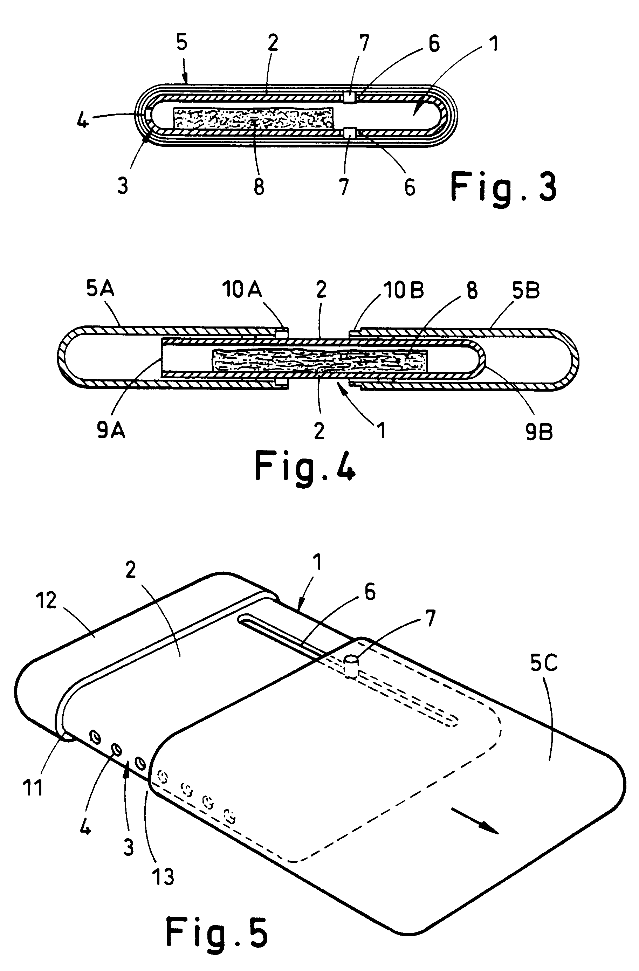

Referring to Fig. 3, the close conformity of shape and size of the casing (5A) to the contained box member (1) is shown. The pins (7) are shown as integral with the casing (5A). Within box member (1) is shown a pellet of a volatile solid material, being a medicament (8). The front edge (3) is curved, giving a lozenge-shaped cross section to the box member.

Referring to Fig. 4, an end edge (9A) of the box member is shown as open, and an alternative arrangement in which an end edge (9B) is closed is also shown. Elements (5A,5B) are shown as being provided with interlockable portions (10A,10B) of a generally known type.

Referring to Fig.5, an alternative form of inhaler is shown, having a box member (1), walls (2), a front edge (3), openings (4) and slot(s) (6) corresponding in form and function to that shown in Fig. 1. The inhaler is provided with only one casing member (5C), having pins (7), and corresponding in form and function to (5B) shown in Fig. 1.

One end (11) of box member (1) is closed and is formed into an integral flange (12), against which end (13) of casing element can butt when the element is in its first, covering, position. Flange (12) and end (13) may be provided with interlocking portions (not shown) similar to those (10A, 10B) shown in Fig. 4.

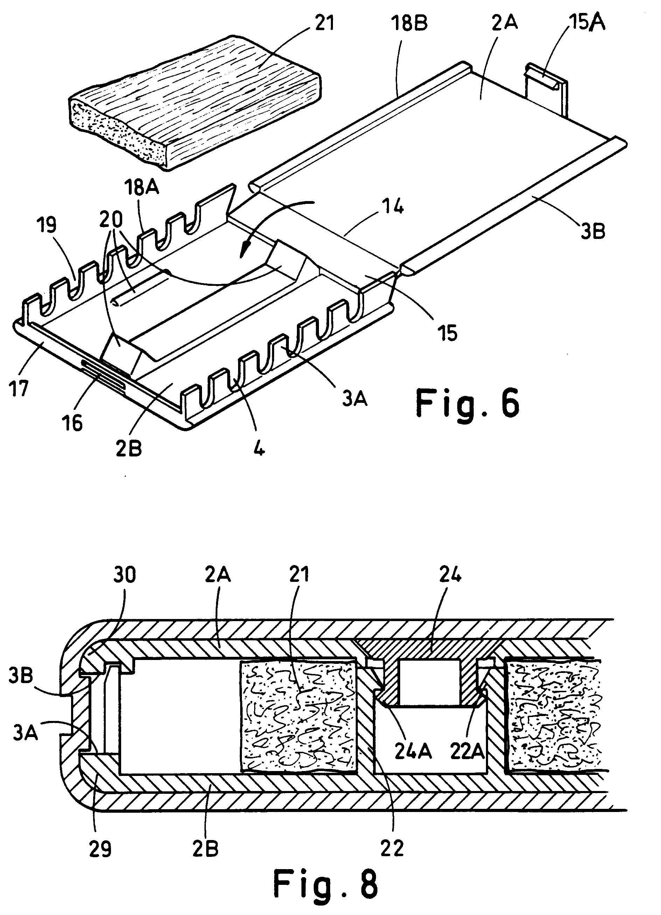

Referring to Fig 6, an alternative form of box member (1) overall is shown, having one of its walls (2A) hinged by a film hinge (14) to an end edge wall (15). The wall (2A) is also provided with a clip (15A) capable of engaging in a snap-fit with a corresponding slot (16) in end edge wall (17) to enable the box member (1) to be closed. The box member (1) has a front edge wall (3) having inhalation openings (4) therein, and a rear edge wall (18) having openings (19) therein for the ingress of air. The front and rear edge walls (3, 18) are formed on closing the box member (1) about the hinge (14) by part edge walls (3A, 3B, 18A, 18B) on walls ((2A, 2B). On wall (2B) are positioning projections (20) which enable correct and secure positioning of a pad (21) of a wicking material impregnated with a volatile material for inhalation.

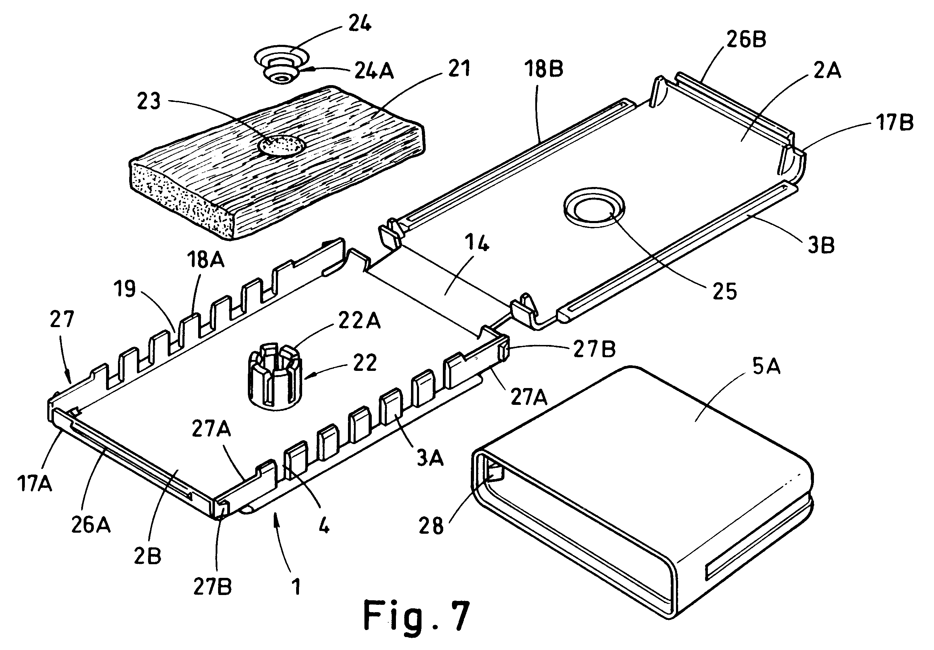

Referring to Fig 7, another alternative form of box member (1) overall is shown. As will be apparent the general construction of this box member is similar to that shown in Fig 6, and common features are numbered correspondingly.

In wall (2B) of box member (1) is formed a socket (22), of generally castellated shape. Socket (22) is positioned and shaped so as to engage with aperture (23) in pad (21) when pad (21) is inserted into box member (1) and thereby retain pad (21) in place. Socket (22) is also provided with teeth (22A) on the free ends of its castellations, which are capable of engaging with a corresponding lip (24A) on a plug (24) which may be inserted through aperture (25) in wall (2A) when box member (1) is folded closed about hinge (14), so as to assist in holding box member (1) closed.

When the box member (1) is folded closed about hinge (14), end walls (17) are formed by the coming together of part end walls (17A, 17B, 17C, 17D). Part end walls (17A and 17B) are held together when the box member (1) is closed by interlocking catch members (26A, 26B).

At each end of the part edge walls (3A, 18A) are end stop members (27) each in the form of a resilient tongue (27A) having at its free end a tooth (27B). In the side wall of casing member (5A) is a corresponding tooth (28). Teeth (27B) are so positioned and shaped so that as the closed box member (1) is inserted into casing (5A) it can ride over corresponding tooth (28) in the side wall of casing (5A), but on attempted removal of closed box member (1) from casing (5) teeth (27B) and (28) engage so as to prevent removal of box member (1) from casing (5A).

Referring to Fig. 8, a partial cross sectional view through an inhaler incorporating the box member illustrated in Fig 7 is shown. The plug (24) has engaged with the socket (22). Edge wall (3) has been formed by engagement of part edge walls (3A, 3B) on closing of the box member (1). The profile of the edge wall (3) includes projecting ridges (29) which engage with corresponding grooves (30) in the edge walls of the casing element (5A).

The method of use of all of the inhalers described above is similar. In use the inhaler is normally supplied to the user with the casing elements (5A,5B,5C) in the first, covering, position, for example as shown in Fig. 2. The elements (5A,5B,5C) may then be slid by hand action in the directions shown by the arrows in Figs. 1 and 5 into the second, uncovered, position, e.g. as shown in Fig. 1, further sliding of the elements (5A,5B,5C) being prevented by the end stopping of the pins (7) against slot ends (6A), or of teeth (27B) against teeth (28). The user may then hold the inhaler in the manner of a mouth organ to inhale air laden with the vapour of volatile material by sucking through openings (4). After use the elements (5A,5B,5C) may be slid in the reverse direction into the first, covering position, e.g. as shown in Fig. 2 to prevent contamination and evaporation of the volatile material.

## Claims

1. An oral inhaler comprising; a box member (1) having two side walls (2) separated by an edge distance less than the length and width dimensions of each side wall (2), and having an edge wall (3) as a front edge which has at least one inhalation opening (4) therein for inhalation therethrough, said box member (1) containing or capable of containing a volatile material (8) for inhalation, the box member (1) having at least one additional opening (6) for the ingress of air; said box member (1) being at least partly contained within a substantially conforming casing (5A,5B,5C) comprised of one or more casing elements (5A) (5B) (5C) which are slideably mounted upon the box member (1); *characterised* in that the said casing element(s) (5A), (5B), (5C) is/are capable of being slid relative to said box member (1) in a direction parallel to the front edge (3) of the box member (1) between a first position in which said opening(s) (4) in the front edge (3) of the box member (1) is/are covered by (a) slideable casing element(s) (5A), (5B), (5C) and into a second position in which the opening(s) (4) is/are uncovered by the slideable casing element(s) (5A), (5B), (5C).

2. An oral inhaler according to claim 1 *characterised* in that the side walls (2) are parallel, planar and generally rectangular in shape.

3. An oral inhaler according to claim 1 or 2 *characterised* in that the edge distance is 0.25 or less of the length dimension.

4. An oral inhaler according to any one of the preceding claims *characterised* in that the box member (1) is tetragonal in shape.

5. An oral inhaler according to any one of the preceding claims *characterised* in that the casing (5A,5B,5C) is tetragonal in shape.

6. An oral inhaler according to any one of the preceding claims *characterised* in that box member (1) is completely contained within the casing (5A,5B,5C) when the casing element(s) (5A,5B,5C) is/are slid into its/their first position, and partly contained when the casing element(s) (5A,5B,5C) is/are slid into its/their second position.

7. An oral inhaler according to any one of the preceding claims *characterised* in that the casing (5C) comprises a single casing element (5C) slideably mounted upon the box member (1).

8. An oral inhaler according to any one of claims 1 to 7 *characterised* in that the casing comprises two casing elements (5A, 5B) slideably mounted on the box member (1).

9. An oral inhaler according to claim 7 or 8 *characterised* in that the two casing elements (5A, 5B) are independently slideable toward opposite ends of the box member (1), so that in their second position the opening(s) (4) in the front wall (3) are located between the two casing elements (5A,5B).

10. An oral inhaler according to any one of the preceding claims, *characterised* in that the volatile material (8) is a solid, as a pellet or within an air permeable container or capsule, or a liquid impregnated on a wicking material or into a solid matrix.

## Patentansprüche

1. Oralinhalationsvorrichtung mit: einem Kastenelement (1) mit zwei Seitenwänden (2), die in einem Randabstand beabstandet sind, der kleiner ist als die Längen- und die Breitenabmessung jeder Seitenwand (2), und mit einer Randwand oder vorderen Wand (3) als vorderer Rand, in der mindestens eine Inhalationsöffnung (4) ausgebildet ist, durch die inhaliert werden kann, wobei das Kastenelement (1) ein flüchtiges Inhalationsmaterial (8) zum Inhalieren enthält oder enthalten kann, das Kastenelement (1) mindestens eine zusätzliche Öffnung (6) aufweist, durch die Luft eindringen kann, das Kastenelement (1) mindestens teilweise in einem im wesentlichan diesem angepaßten Gehäuse (5A, 5B, 5C) angeordnet ist, das ein oder mehrere auf dem Kastenelement (1) verschiebbar angeordnete(s) Gehäuseelement(e) (5A), (5B), (5C) aufweist, **dadurch gekennzeichnet**, **daß** das/die Gehäuseelement(e) relativ zum Kastenelement (1) parallel zum vorderen Rand (3) des Kasterelements (1) verschiebbar ist/sind zwischen einer ersten Position, bei der die Öffnung(en) (4) im vorderen Rand (3) des Kastenelements (1) durch ein verschiebbares Gehäuseelement bzw. durch verschiebbare Gehäuseelemente (5A), (5B), (5C) abgedeckt ist/sind, und einer zweiten Position, bei der die Öffnung(en) (4) durch das/die verschiebbaren Gehäuseelement(e) (5A), (5B), (5C) nicht abgedeckt ist/sind.

2. Oralinhalationsvorrichtung nach Anspruch 1, **dadurch gekennzeichnet**, **daß** die Seitenwände (2) parallel, planar und allgemein rechtwinklig ausgebildet sind.

3. Oralinhalationsvorrichtung nach Anspruch 1 oder 2, **dadurch gekennzeichnet**, **daß** der Randabstand höchstens so groß ist wie die 0,25-fache Längenabmessung.

4. Oralinhalationsvorrichtung nach einem der vorangehenden Ansprüche, **dadurch gekennzeichnet**, **daß** das Kastenelement (1) eine tetragonale Form aufweist.

5. Oralinhalationsvorrichtung nach einem der vorangehenden Ansprüche, **dadurch gekennzeichnet**, **daß** das Gehäuse (5A, 5B, 5C) eine tetragonale Form aufweist.

6. Oralinhalationsvorrichtung nach einem der vorangehenden Ansprüche, **dadurch gekennzeichnet**, **daß** das Kastenelement (1) vollständig im Gehäuse (5A, 5B, 5C) angeordnet ist, wenn das/die Gehäuseelement(e) (5A, 5B, 5C) auf seine/ihre erste Position eingestellt ist/sind, und teilweise im Gehäuse angeordnet ist, wenn das/die Gehäuseelement(e) (5A, 5B, 5C) auf seine/ihre zweite Position eingestellt ist/sind.

7. Oralinhalationsvorrichtung nach einem der vorangehenden Ansprüche, **dadurch gekennzeichnet**, **daß** das Gehäuse (5C) ein auf dem Kastenelement (1) verschiebbar angeordnetes einzelnes Gehäuseelement (5C) aufweist.

8. Oralinhalationsvorrichtung nach einem der Ansprüche 1 bis 7, **dadurch gekennzeichnet**, **daß** das Gehäuse zwei auf dem Kastenelement (1) verschiebbar angeordnete Gehäuseelemente (5A, 5B) aufweist.

9. Oralinhalationsvorrichtung nach Anspruch 7 oder 8, **dadurch gekennzeichnet**, **daß** die beiden Gehäuseelemente (5A, 5B) unabhängig zu entgegengesetzten Enden des Kastenelements (1) verschiebbar sind, so daß, wenn sie auf ihre zweite Position eingestellt sind, die Öffnung(en) (4) in der Vorerwand (3) zwischen den beiden Gehäuseelementen (5A, 5B) angeordnet ist/sind.

10. Oralinhalationsvorrichtung nach einem der vorangehenden Ansprüche, **dadurch gekennzeichnet**, **daß** das flüchtige Material (8) ein Feststoff ist, wie beispielsweise ein Pellet, in einem luftdurchlässigen Behälter bzw. einer luftdurchlässigen Kapsel enthalten ist, oder eine auf einem Docht- oder Gazematerial oder in einer Festkörpermatrix imprägnierte Flüssigkeit ist.

## Revendications

1. Inhalateur oral comprenant : un élément en forme de boîte (1) comportant deux parois latérales (2) séparées par une distance au niveau des bords inférieure aux dimensions en longueur et en largeur de chaque paroi latérale (2) et possédant une paroi de bord (3) en tant que bord avant, qui possède au moins une ouverture d'inhalation (4) permettant une inhalation, ledit élément en forme de boîte (1) contenant ou étant à même de contenir une substance volatile (8) pour l'inhalation, l'élément en forme de boîte (1) possédant au moins une ouverture supplémentaire (6) pour la pénétration de l'air, ledit élément en forme de boîte (1) étant au moins en partie contenu à l'intérieur d'un boîtier ayant une forme essentiellement adaptée (5A, 5B, 5C) constitué par un ou plusieurs éléments de boîtier (5A) (5B) (5C) qui sont montés de manière à pouvoir glisser sur l'élément en forme de boîte (1), caractérisé en ce que le ou lesdits éléments de boîtier (5A), (5B), (5C) est/sont à même de glisser par rapport audit élément en forme de boîte (1) dans une direction parallèle au bord avant (3) de l'élément en forme de boîte (1) entre une première position, dans laquelle ladite ou lesdites ouvertures (4) aménagées dans le bord avant (3) de l'élément en forme de boîte (1) est/sont couverte(s) par un ou des éléments de boîtier coulissant (5A), (5B), (5C) et peuvent être amenées dans une seconde position, dans laquelle la ou les ouvertures (4) est/sont recouverte(s) par le ou les éléments de boîtier coulissant (5A), (5B), (5C).

2. Inhalateur oral selon la revendication 1, caractérisé en ce que les parois latérales (2) sont parallèles, planes et de forme générale rectangulaire.

3. Inhalateur oral selon la revendication 1 ou 2, caractérisé en ce que la distance entre bords est égale à 0,25 de la dimension en longueur ou moins.

4. Inhalateur oral selon l'une quelconque des revendications précédentes, caractérisé en ce que l'élément en forme de boîte (1) a une forme de quadrilatère.

5. Inhalateur oral selon l'une quelconque des revendications précédentes, caractérisé en ce que le boîtier (5A, 5B, 5C) a une forme de quadrilatère.

6. Inhalateur oral selon l'une quelconque des revendications précédentes, caractérisé en ce que l'élément en forme de boîte (1) est entièrement logé à l'intérieur du boîtier (5A, 5B, 5C) lorsque le ou les éléments de boîtier (5A, 5B, 5C) est/sont amené(s) par glissement dans sa/leur première position, est logé partiellement dans le boîtier lorsque le ou les éléments de boîtier (5A, 5B, 5C) est/sont amené(s) par glissement dans sa/leur seconde position.

7. Inhalateur oral selon l'une quelconque des revendications précédentes, caractérisé en ce que le boîtier (5C) comprend un seul élément de boîtier (5C) monté de manière à pouvoir glisser sur l'élément en forme de boîte (1).

8. Inhalateur oral selon l'une quelconque des revendications 1 à 7, caractérisé en ce que le boîtier comprend deux éléments de boîtier (5A, 5B) montés de manière à pouvoir glisser sur l'élément de boîtier (1).

9. Inhalateur oral selon la revendication 7 ou 8, caractérisé en ce que les deux éléments de boîtier (5A, 5B) peuvent glisser de façon indépendante en direction d'extrémités opposées de l'élément en forme de boîte (1) de sorte que, dans leur seconde position, la ou les ouvertures (4) aménagée(s) dans la paroi avant (3) est/sont située(s) entre les deux éléments de boîtier (5A, 5B).

10. Inhalateur oral selon l'une quelconque des revendications précédentes, caractérisé en ce que la substance volatile (8) est un solide, telle qu'une pastille, ou est placée dans un récipient ou une capsule perméable à l'air, ou est un liquide imprégnant un matériau à effet de mèche ou dans une matrice solide.
